# EUROPEAN PATENT APPLICATION

(11) **EP 4 361 772 A1**
(43) Date of publication of application: **01.05.2024**
(21) Application number: 22204747.4
(22) Date of filing: 31.10.2022
(51) Int. Cl.: G06F 3/01, G06F 3/0481, G06F 3/04842, G06F 3/04895, G06F 9/451, G16H 10/00, G06F 11/34

(54) **DETECTION OF EFFICIENT USE OF DEVICE FEATURES**

(71) Applicant: Koninklijke Philips N.V., 5656 AG Eindhoven (NL)
(72) Inventor: CHAKRABARTI, Biswaroop, Eindhoven (NL); VAN EE, Raymond, Eindhoven (NL); JELFS, Sam Martin, 5656AG Eindhoven (NL); MENDOZA, Jose Luis Diaz, 5656AG Eindhoven (NL); ANAND, Shreya, Eindhoven (NL); RUTJES, Heleen, Eindhoven (NL)
(74) Representative: Philips Intellectual Property & Standards

(57) **Abstract**

The present invention relates to a system for characterizing engagement of a user with a medical device. The system comprises a logging module for logging usage data regarding a feature utilization time of the user of a feature of the medical device, and an evaluation module for evaluating the logged usage data and for determining a variance in the feature utilization time. The system further comprises a stress measurement module for determining a stress level of the user during utilization of the feature of the medical device, and an analyzation module for deriving an efficiency of usage of the feature of the medical device by the user from the variance in the feature utilization time and the determined stress level of the user.

## Description

### FIELD OF THE INVENTION

The present invention relates to a system for characterizing engagement of a user with a medical device and a method for characterizing engagement of a user with a medical device.

### BACKGROUND OF THE INVENTION

A user of a medical device or system, like, for example, an ultrasound imaging device or an endoscopic system, may encounter different features of the device during interacting with the medical device. Some features may be unintuitive and/or difficult to use and may lead to stress of the user, who may not take properly advantage of the respective feature. This sort of under usage may limit the utilization of the feature set of the medical device and might be difficult to detect, especially if logging information of the device is sparse.

It is therefore desirable to characterize the engagement of end-users of a medical device like an ultrasonography device. Whether the device features are used in an efficient way can contribute to the engagement characterization. Device logs can be a resource for efficiency analysis. However, depending on the imaging modality and the logging settings, the logs can be sparse/absent or may not lead directly to efficiency/feature utilization detection, especially on a per user/per procedure type basis.

The inventors of the present invention have thus found that it would be advantageous to have a system for characterizing engagement of a user with a medical device that does not suffer from the above-mentioned drawbacks. In addition, a frustrating user experience regarding a particular feature may lead to stress and it would be beneficial to correlate stress with feature usage to optimize the workflow by modifying how the feature is presented to the user.

### SUMMARY OF THE INVENTION

It is an object of the present invention to provide an improved system for characterizing engagement of a user with a medical device that determines whether a medical device is used efficiently by a user of the medical device. A system and a method is proposed to utilize possibly sparse logging data and stress characterization to derive a fine-grained characterization of device feature utilization.

The object of the present invention is solved by the subject matter of the independent claims, wherein further embodiments are incorporated in the dependent claims.

The described embodiments similarly pertain the system for characterizing engagement of a user with a medical device and the method for characterizing engagement of a user with a medical device. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a system for characterizing engagement of a user with a medical device. The system comprises a logging module, configured for logging usage data regarding a feature utilization time of the user of a feature of the medical device, and an evaluation module, configured for evaluating the logged usage data and for determining a variance in the feature utilization time. The system further comprises a stress measurement module, configured for determining a stress level of the user during utilization of the feature of the medical device, and an analyzation module, configured for deriving an efficiency of usage of the feature of the medical device by the user from the variance in the feature utilization time and the determined stress level of the user.

Thus, the proposed system comprises a module, preferably a software module, operating at a medical device or system and logging a set of information that can comprise an identifier for a device feature or an atomic UI element and the time spent by a user while operating the device or system. In addition, the system comprises a statistical module as evaluation module operating on the logged information to measure variance in the feature utilization time of the respective feature. Further, the system comprises a stress measurement module that determines a stress level of the user while operating the respective feature of the medical device. Stress can be measured, for example, via determining various physiological parameters of the user, like galvanic skin response, heart rate variability, eye movement tracking, etc. An objective measure of the stress level of the user can be computed from these measurements. In addition or alternatively, in case the measurement of these parameters is not possible, a machine learning model can be trained to use as input the users' interaction pattern with the medical device, output the putative stress level of the user based on this input. Alternatively, direct feedback to the system provided by the user about their experience while using the medical device and/or their perceived stress can be used to determine the stress level of the user. There may be options to provide feedback at different levels of detail. The analyzation module correlates the determined variance in the feature utilization time and the measured stress level of the user while using the feature and derives an efficiency of usage of the respective feature. With this information it is possible to identify features that are unintuitive or difficult to use based on the temporal analysis of feature usage, and alternatively unfinished processes or workflows. The feature usage can be correlated to the stress level of the user, especially even if traditional stress measurement methods based on physiological parameters cannot be employed.

Thus, the system according to the invention may infer efficiency and ease of use of a feature of the medical device, and additionally patterns of utilization of a feature may be determined. Variance in time spent per procedure or feature can have many causes, for example, some departments may have other workflows than other departments, patient cases may differ, there might be seasonal differences, or time on day may have an influence on the length of the procedure, one operator may structurally spend more time than another operator, and many offer things. When statistically correcting for these known sources of variation in the utilization time, what remains is a residual variance. The inventors of the present invention found that the complexity of a feature can be expressed in the size of this residual variance. For non-intuitive or complex features, the residual variance is larger than for more straight-forward or easily usable features, because when higher complexity is involved, learning and making use of the feature in its intended and efficient way cannot be presumed. Inconsistencies in the time spent in a single procedure can thus indicate varying skill and understanding of the user, non-intuitiveness and/or inconsistencies in the user interface, or variable complexities for conditions where use of the respective feature is indicated. Some features may be inherently complex and require long time to become familiar with the feature, while some features may be inherently simple to use. However, the usage of certain features may be more involved for one clinical condition being investigated while relatively simple for other clinical conditions.

Varying skills of the user and non-intuitiveness of the user interface can be resolved from the variable complexities by analyzing the clinical indications, i.e. the procedure prescriptions and/or the diagnosed conditions to check if the variance persists.

The evaluation module as statistical analysis module can identify aberrant patterns of device usage. The procedure can be as follows. A feature A is considered, and the distribution of duration of this feature's usage is created. A list of known sources of variances like department level, patient level, operator level, seasonal level, etc. is provided and it can be correct for those effects by statistically control, for example. What remains is the residual variance of usage of this feature, which is called σA. The same is done for feature B, resulting in σB, and so on, for all available features of the medical device. Then, the distribution of the different σ's is considered, representing a summary and comparison of all features. Of that final distribution, the tail or tails can be considered to be candidates for further exploration, as they most likely refer to complex and non-intuitive features.

On that final distribution, various statistical metrics can be employed, for example standard deviation of the time spent, possibly normalized as the coefficient of variation. A threshold value may be preselected to characterize a feature as having an unacceptably high variation in usage time. Standardized moments of different degrees, e.g., skewness and kurtosis, can be calculated for the distribution of the time spent using the feature to further characterize the distribution. Threshold values of the normalized measures can indicate aberrant feature usage. Other appropriate metrics can be envisaged, e.g., matching with templated patterns of time spent, correlation of the time spent and/or usage of a feature with the load, e.g., the number of studies in the worklist, time of day etc. Temporal analysis of feature usage to check under-usage in high load situations may be performed.

Thus, identification of unintuitive and stress-inducing device features and user interface elements can improve any medical device or system that the user like a physician or any other clinical personnel interacts with.

In an embodiment of the invention, the system comprises an output module configured for displaying the derived efficiency of usage of the feature of the medical device to the user and/or to administrative staff of the medical device. This optional module can display level-appropriate recommendations to the user and/or can display summarized feature utilization data to the administrative staff of the device. Thus, necessary inputs to the user can be provided to remedy the feature usage difficulties, by, for example, training videos, feature usage walkthrough, tips, etc. Alternatively or in addition, summarized information can be provided to the hospital administrator or a person in charge of managing the workflows related to the medical device.

In an embodiment of the invention, the system comprises a display unit configured for displaying instructions to the user how to utilize the feature of the medical device. This display unit can preferably be part of the output module.

In an embodiment of the invention, the logging module is configured for logging usage data regarding a number of incomplete attempts and a number of complete attempts the user utilizes the feature of the medical device. These data can help to derive a difficulty of usage and a complexity of the respective feature. A high relative number of incomplete attempts while using a feature in relation to the number of completed processes, workflows, or tasks where the feature is involved can indicate an inefficient use of the feature due to a high complexity.

In an embodiment of the invention, the stress measurement module is configured to measure the stress level of the user via a measurement of at least one physiological parameter, and the at least one physiological parameter is galvanic skin response, heart rate, heart rate variability, blood, pressure, facial thermal variation, and/or eye movement tracking.

For the stress level analyzer module, when stress measurement is practicable and acceptable to the user, various physiological measurements can be obtained. For example, possible inputs may be galvanic skin response, heart rate and/or and heart rate variability, blood pressure, facial thermal variation, eye movement tracking, etc. An objective stress measure can be computed from such measurements.

In an embodiment of the invention, the stress measurement module is configured to measure the stress level of the user via an analysis of an interaction pattern of the user with the medical device, and/or the stress measurement module is configured to measure the stress level of the user via direct feedback of the user regarding an experience of the user while using the medical device. In case the institution like the hospital or the user may not consent to these measurements of physiological parameters during device usage as they may be perceived as invasive and/or time-consuming, a machine learning model can be constructed with user interaction patterns with the medical device. For example, keystrokes, pointing device movement, dwell times on UI elements, or traversal pattern within the workflow - whether the user goes back to a previously visited element - can be labelled with objective stress level measurement that are measured through physiological measurement described above and computed. Thus, a surrogate stress measurement is possible with the user's interaction pattern with the system used as input for the machine learning model and the stress level of the user provided as output of the model. The stress level of the user thus measured can be correlated with procedure labels or a respective feature to check if the feature leads to stress and/or if the stress level of the user impacts performance with efficiency of the procedure. Such a model can be deployed for stress characterization and output the putative stress level. In addition or alternatively, the users can provide direct feedback to the system about their experience while using the system and/or their perceived stress. There can be options to provide feedback at different levels of detail.

In an embodiment of the invention, the logging module is configured for logging usage data regarding an identifier for the feature, a role and/or position of the feature in a clinical workflow, a diagnosed medical condition of a patient treated with the medical device, and/or an identifier of the user. Thus, optionally, other information may be logged, including but not limited to role/position of the feature in a broader clinically meaningful workflow, medical conditions diagnosed and/or indications for the investigating procedure, or a possibly anonymized identifier for the user, etc.

In an embodiment of the invention, the analyzation module is configured for analyzing a use of the feature of the medical device with respect to a set of features of the medical device, and/or the analyzation module is configured for determining a usage pattern of the feature of the medical device. Optional co-analysis of available features in an imaging modality, their utilization and procedure prescription and final report can reveal under-utilization of the feature set. Feature utilization patterns and procedures can reveal gaps in the feature set of the device/system, and temporal analysis of the feature set utilization can show the ease of use and whether the feature is required or optional.

If under-usage or an aberrant usage pattern for a particular feature is detected, the user may benefit from training and tips. However, presentation of such aids is preferably tailored to the user needs. For example, when the user is working during a high-load situation, an unobtrusive non-modal tip may be presented and at a time when the user is relatively relaxed, a more detailed walk-through can be offered. If the procedure is inherently complex or may lead to stress, the procedure code and/or the diagnosis can be considered to tune the contents and delivery mode of the feedback so that the message is not disruptive but can be acted upon.

If the temporal analysis of feature usage results in under-usage of certain features in high load situations, it can be used as an input to a planning or scheduling system so that studies with indications that may benefit from the feature are scheduled during a less stressful period. Differing presentation and scheduling can be part of research on the usability of said features.

Keeping track of the traversal path to locate a feature in the user interface of the medical device, and suggesting a shorter way, if present can improve user experience. Often used features can be suggested depending on usage context like a bookmark, for example. Further, presenting feature usage gaps to the administrator of the device in a summarized way with the aid of a dashboard may support efficient use of the medical device in the future. Feedback retrieval can be part of the process to improve the experience. Feedback retrieval can be just after the procedure, or can be limited to low stress and/or low throughput situations.

In an embodiment of the invention, the system comprises an anonymizer data processing module configured for extracting an anonymized procedure indication, a patient report and/or patient parameters. Thus, sensitive patient data can be used for statistical evaluation while ensuring data protection and privacy protection of the patient.

In an embodiment of the invention, the evaluation module is configured for correcting the determined variance in the feature utilization time for effects due to department workflow, patient case, operator level, and/or seasonal differences. This allows an improved determination of the residual variance in the feature utilization time as known sources for systematic errors can be eliminated.

In an embodiment of the invention, the system comprises an eye tracking module configured for tracking an eye of the user and configured for determining whether the user recognizes information provided by the medical device. Thus, it can be determined whether the user efficiently notice important information in the system of the medical device.

According to another aspect of the invention, there is provided a medical device comprising the system for characterizing engagement of a user with the medical device according to any of the preceding embodiments.

According to another aspect of the invention, there is provided a computer-implemented method for characterizing engagement of a user with a medical device, the method comprising the steps of logging usage data regarding a feature utilization time of the user of a feature of the medical device, evaluating the logged usage data and determining a variance in the feature utilization time, determining a stress level of the user during utilization of the feature of the medical device, and deriving an efficiency of usage of the feature of the medical device by the user from the variance in the feature utilization time and the determined stress level of the user.

According to another aspect of the invention, there is provided a computer program comprising instructions which, when the program is executed by a computer, cause the computer to carry out the steps of the method according to any of the preceding embodiments.

According to another aspect of the invention, there is provided a computer-readable storage medium comprising instructions which, when executed by a computer, cause the computer to carry out the steps of the method according to any of the preceding embodiments.
Thus, the benefits provided by any of the above aspects equally apply to all of the other aspects and vice versa.

In a gist, the invention relates to a system for characterizing engagement of a user with a medical device. The system comprises a logging module for logging usage data regarding a feature utilization time of the user of a feature of the medical device, and an evaluation module for evaluating the logged usage data and for determining a variance in the feature utilization time. The system further comprises a stress measurement module for determining a stress level of the user during utilization of the feature of the medical device, and an analyzation module for deriving an efficiency of usage of the feature of the medical device by the user from the variance in the feature utilization time and the determined stress level of the user.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic setup of a system for characterizing engagement of a user with a medical device according to an embodiment of the invention.
Fig. 2 shows a schematic setup of a system for characterizing engagement of a user with a medical device according to another embodiment of the invention.
Fig. 3 shows a block diagram of a method for characterizing engagement of a user with a medical device according to another embodiment of the invention.

### DETAILED DESCRIPTION OF EMBODIMENTS

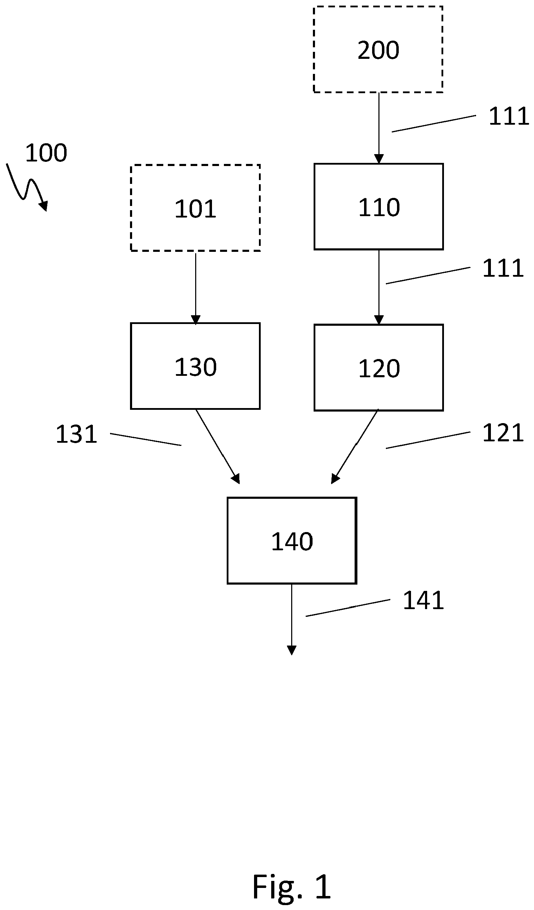

Fig. 1 shows a schematic setup of a system 100 for characterizing engagement of a user 101 with a medical device 200 according to an embodiment of the invention. Usage data 111 of a feature of the medical device 200 are transmitted from the medical device 200 to the logging module 110 of the system 100. The logged usage data 111 are forwarded from the logging module 110 to the evaluation module 120, where a variance 121 in feature utilization time is determined and provided to the analyzation module 140. The stress measurement module 130 receives data indicative of a stress level of the user form the user 101, and determines a stress level 131 of the user 101, which is provided to the analyzation module 140. The analyzation module 140 derives an efficiency of usage 141 of the feature of the medical device 200 by the user 101 from the variance 121 in the feature utilization time and the determined stress level 131 of the user 101.

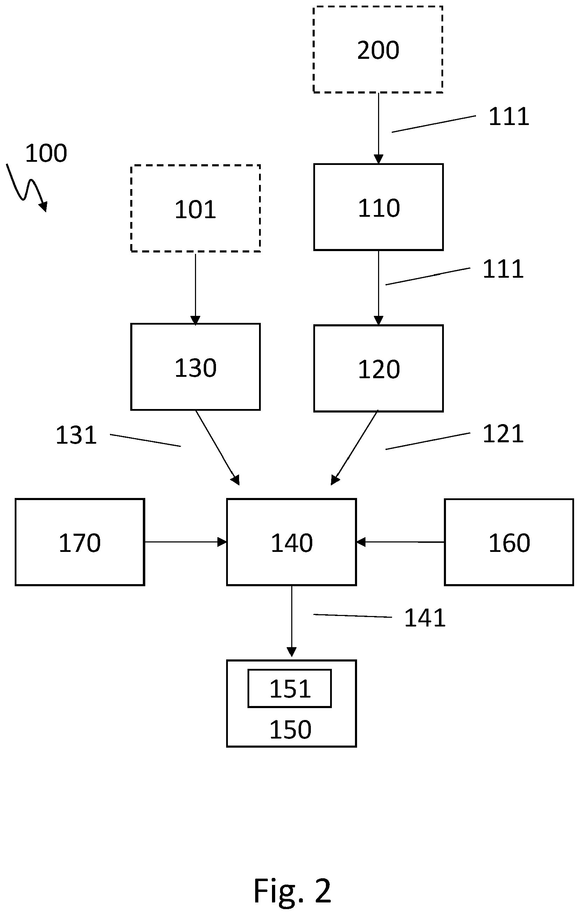

Fig. 2 shows a schematic setup of a system 100 for characterizing engagement of a user 101 with a medical device 200 according to another embodiment of the invention. In addition to the system already described with respect to Fig. 1, Fig. 2 shows an output module 150 with a display unit 151, which receives the determined efficiency of usage 141 from the analyzation module 140. Further, an anonymizer data processing module 160 is shown, which provides patient data from a patient in an anonymized form to the analyzation module 140. An eye tracking module 170 can track an eye of the user 101 and control, whether the user 101 recognizes information provided by the medical device 200 and/or the output module 150.

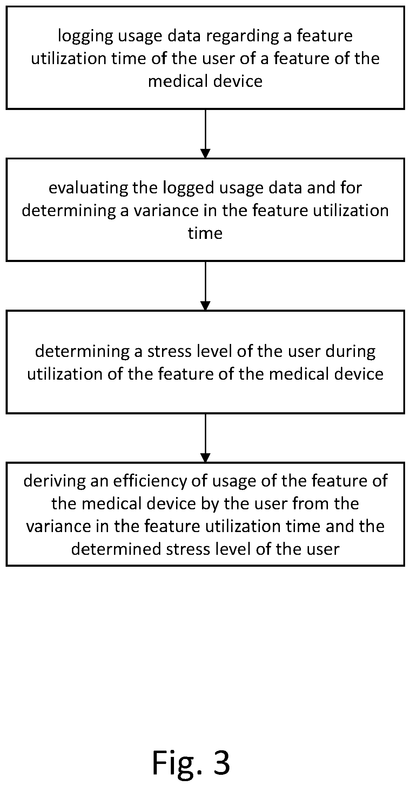

Fig. 3 shows a block diagram of a method for characterizing engagement of a user 101 with a medical device 200 according to another embodiment of the invention. The method comprises the steps of logging usage data 111 regarding a feature utilization time of the user 101 of a feature of the medical device 200, evaluating the logged usage data 111 and determining a variance 121 in the feature utilization time, determining a stress level 131 of the user 101 during utilization of the feature of the medical device 200, and deriving an efficiency of usage 141 of the feature of the medical device 200 by the user 101 from the variance 121 in the feature utilization time and the determined stress level 131 of the user 101.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: system
- 101: user
- 110: logging module
- 111: usage data
- 120: evaluation module
- 121: variance in feature utilization time
- 130: stress measurement module
- 131: stress level
- 140: analyzation module
- 141: efficiency of usage
- 150: output module
- 151: display unit
- 160: anonymizer data processing module
- 170: eye tracking module
- 200: medical device

## Claims

1. A system (100) for characterizing engagement of a user with a medical device, the system comprising:
a logging module (110), configured for logging usage data (111) regarding a feature utilization time of the user (101) of a feature of the medical device (200);
an evaluation module (120), configured for evaluating the logged usage data (111) and for determining a variance (121) in the feature utilization time;
a stress measurement module (130), configured for determining a stress level (131) of the user (101) during utilization of the feature of the medical device (200); and
an analyzation module (140), configured for deriving an efficiency of usage (141) of the feature of the medical device (200) by the user (101) from the variance (121) in the feature utilization time and the determined stress level (131) of the user (101).

2. The system (100) according to claim 1, wherein the system (100) comprises an output module (150) configured for displaying the derived efficiency of usage (141) of the feature of the medical device (200) to the user (101) and/or to administrative staff of the medical device (200).

3. The system (100) according to any of the preceding claims, wherein the system (100) comprises a display unit (151) configured for displaying instructions to the user (101) how to utilize the feature of the medical device (200).

4. The system (100) according to any of the preceding claims, wherein the logging module (110) is configured for logging usage data (111) regarding a number of incomplete attempts and a number of complete attempts the user (101) utilizes the feature of the medical device (200).

5. The system (100) according to any of the preceding claims, wherein the stress measurement module (130) is configured to measure the stress level (131) of the user (101) via a measurement of at least one physiological parameter, and wherein the at least one physiological parameter is galvanic skin response, heart rate, heart rate variability, blood, pressure, facial thermal variation, and/or eye movement tracking.

6. The system (100) according to any of the preceding claims, wherein the stress measurement module (130) is configured to measure the stress level (131) of the user (101) via an analysis of an interaction pattern of the user (101) with the medical device (200), and/or wherein the stress measurement module (130) is configured to measure the stress level (131) of the user (101) via direct feedback of the user regarding an experience of the user while using the medical device (200).

7. The system (100) according to any of the preceding claims, wherein the logging module (110) is configured for logging usage data (111) regarding an identifier for the feature, a role and/or position of the feature in a clinical workflow, a diagnosed medical condition of a patient treated with the medical device (200), and/or an identifier of the user (101).

8. The system (100) according to any of the preceding claims, wherein the analyzation module (140) is configured for analyzing a use of the feature of the medical device (200) with respect to a set of features of the medical device (200), and/or wherein the analyzation module (140) is configured for determining a usage pattern of the feature of the medical device (200).

9. The system (100) according to any of the preceding claims, wherein the system (100) comprises an anonymizer data processing module (160) configured for extracting an anonymized procedure indication, a patient report and/or patient parameters.

10. The system (100) according to any of the preceding claims, wherein the evaluation module (120) is configured for correcting the determined variance (121) in the feature utilization time for effects due to department workflow, patient case, operator level, and/or seasonal differences.

11. The system (100) according to any of the preceding claims, wherein the system (100) comprises an eye tracking module (170) configured for tracking an eye of the user (101) and configured for determining whether the user (101) recognizes information provided by the medical device (200).

12. A medical device (200) comprising the system (100) for characterizing engagement of a user (101) with the medical device (200) according to any of claims 1 to 11.

13. A computer-implemented method for characterizing engagement of a user (101) with a medical device (200), the method comprising the steps of:
logging usage data (111) regarding a feature utilization time of the user (101) of a feature of the medical device (200);
evaluating the logged usage data (111) and determining a variance (121) in the feature utilization time;
determining a stress level (131) of the user (101) during utilization of the feature of the medical device (200); and
deriving an efficiency of usage (141) of the feature of the medical device (200) by the user (101) from the variance (121) in the feature utilization time and the determined stress level (131) of the user (101).

14. A computer program comprising instructions which, when the program is executed by a computer, cause the computer to carry out the steps of the method according to claim 13.

15. A computer-readable storage medium comprising instructions which, when executed by a computer, cause the computer to carry out the steps of the method according to claim 13.
